# EUROPEAN PATENT SPECIFICATION

(11) **EP 1 628 911 B1**
(45) Date of publication and mention of the grant of the patent: **07.12.2011**
(21) Application number: 04751935.0
(22) Date of filing: 11.05.2004
(51) Int. Cl.: C01B 11/02, B01J 4/00, B01J 4/02, A61L 2/20

(54) **PREPARATION OF AND DISPENSING CHLORINE DIOXIDE**
HERSTELLUNG UND SPENDEN VON CHLORDIOXID
SYSTEME DE PRODUCTION ET DISPERSION DE BIOXYDE DE CHLORE

(30) Priority: 12.05.2003 US 320188 P; 11.05.2004 US 709517
(43) Date of publication of application: 01.03.2006
(60) Divisional application: 10175173.3
(73) Proprietor: Diversey, Inc., Sturtevant, WI 53177 (US)
(72) Inventor: BOBER, Andrew M., c/o JohnsonDiversey, Inc., Sturtevant, WI 53177-0902 (US); CRAWFORD, Charles, c/o JohnsonDiversey, Inc., Sturtevant, WI 53177-0902 (US); GRINSTEAD, Dale E., c/o JohnsonDiversey, Inc., Sturtevant, WI 53177-0902 (US); NISHIZAWA, Masahiro, c/o JohnsonDiversey, Inc., Sturtevant, WI 53177-0902 (US); ROACH, Kenneth J., c/o JohnsonDiversey, Inc., Sturtevant, WI 53177-0902 (US); ROUILLARD, Carol Anne, c/o JohnsonDiversey, Inc., Sturtevant, WI 53177-0902 (US); WHITEHEAD, James H., c/o JohnsonDiversey, Inc., Sturtevant, WI 53177-0902 (US); WRIGHT, William B., c/o JohnsonDiversey, Inc., Sturtevant, WI 53177-0902 (US)
(74) Representative: Ruschke, Hans Edvard
(86) International application number: PCT/US2004/014787
(87) International publication number: WO 2004/101432

(56) References cited:
- WO-A-02/23993
- DE-U- 29 924 373
- GB-A- 2 128 900
- US-A- 4 104 190
- US-A- 5 207 642
- US-A- 5 389 384
- US-A1- 2002 000 225
- US-A1- 2002 161 344
- US-A1- 2003 080 317
- US-B1- 6 231 830
- US-B1- 6 272 879
- US-B1- 6 302 855

## Description

### FIELD OF THE INVENTION

The Invention relates to a process for the production of chlorine dioxide. More specifically the invention relates to a quick and effective method for producing chlorine dioxide without the need for a generator.

### BACKGROUND OF THE INVENTION

Chlorine dioxide has long been recognized as a preferred biocide. It is well known to be effective against a wide spectrum of organism. In spite of this, the use of chlorine dioxide as a biocide has heretofore been limited. Chlorine dioxide is a gas and aqueous solutions of it are inherently unstable. Chlorine dioxide readily volatilizes, that is it readily migrates from solution to the gas phase, unless stored in a closed vessel with no headspace. Moreover, chlorine dioxide is subject to photochemical decomposition and subject to chemical decomposition through disproportionation. The net result is that chlorine dioxide solutions have a relatively short shelf life. To compensate for this chlorine dioxide is produced from relatively stable precursor species at the end use facilities. Chlorine dioxide production at end use facilities has heretofore required either a generator to produce chlorine dioxide solutions or a relatively long reaction time to produce chlorine dioxide from the generatorless systems heretofore known.

The generator based systems are systems which use some mechanical or electrical element to facilitate or control the rate of production of chlorine dioxide. Generators fall into two broad categories: chemical and electrochemical. Typically electrochemical generators fall into two categories, those that oxidize a chlorite ion and those that reduce a chlorate ion. All generator based systems produce relatively high concentrations of chlorine dioxide which must be diluted to give use strength solutions. The safety concerns associated with concentrated solutions of chlorine dioxide are well known. Most generators incorporate elaborate safety systems in an attempt to reduce the risk associated with producing, storing and handling these highly concentrated solutions, contributing significantly to the overall cost. The total cost of these generators, including operation and maintenance costs, have limited their application.

Generatorless systems for producing chlorine dioxide are known, however these systems generally require relatively long reaction times (hours) to produce solutions of chlorine dioxide. As with the concentrated chlorine dioxide solutions produced by the generators, these solutions may require dilution to give use strength solutions. The time constraints associated with these systems have limited their application.

Two recent patents have attempted to address the short comings of the current methods of generating chlorine dioxide. However, both of these (Madray, US Patent No. 6,231,830 and Hei et al., US Patent No 6,663,902) require relatively high concentrations of sodium chlorite and other reactants and still require relatively long reaction times.

Madray U.S. Patent No. 6,231,830 uses an alkali metal chlorite with a alkali metal iodide to produce chlorine dioxide. The patent teaches the need for a buffering agent to maintain the pH above 6.2 and claims a minimum of 300 ppm of the chlorite solution. Madray further requires relatively long reaction times in order to have an effective level of chlorine dioxide produced.

Hei et Al. U.S. Patent No. 6,663,902 uses an iodo-compound and source of chlorite ions to produce chlorine dioxide. The patent teaches the need for long reaction times and very high levels of chlorite and the iodo-compound in order to have effective amounts of chlorine dioxide produced.

US 2003/0080317 A1 discloses a massive body comprising a metal chlorite, an acid source and a source of free halogen, said massive body being such that when it is added to liquid water, it will produce a solution of chlorine dioxide and free halogen.

US 4,104, 190 discloses a method of generating chlorine dioxide in an aqueous liquid containing an alkali metal or alkaline earth metal chlorite by adding to the aqueous liquid a water-soluble solid composition containing a chlorine-releasing component.

Chlorine dioxide 18 an excellent sanitizer and disinfectant but without the proper method of production there are just too many limitations to allow for a wide variety of uses. The fact of the matter is, there still remains a need to produce a fast, safe and effective system for the production and use of chlorine dioxide as a disinfectant and sanitizer.

### SUMMARY OF THE INVENTION

The invention discloses a composition that efficiently produces chlorine dioxide in adequate amounts to have the desired biocidal activity while reducing any safety issues and minimizing the loss of chlorine dioxide through volatilization. The invention encompasses the use of a chlorite in an amount of less than 100 ppm, an activator, a chloride salt, a solvent and a diluent.

The current invention allows for the production of chlorine dioxide on site which will eliminate any issue as to loss of the chlorine dioxide reducing the effectiveness of the composition and also reduces any volatility and safety issues associated with chlorine dioxide production through most current generators. Further the invention allows for the rapid, safe and efficient production of chlorine dioxide while overcoming the cost and safety issues of the generator systems and the slow production rates and safety issues typically associated with generatorless systems. The invention allows for the reaction time to be reduced to a low level so that the invention can be used without long delays while still not being instantaneous, allowing the invention to be used *in situ* which eliminates the loss of chlorine dioxide and loss of effectiveness.

### BRIEF DESCRIPTION OF THE DRAWINGS

FIG. 1 is a perspective view of one embodiment of a dispenser and cart.
FIG. 2 is an exploded view of the embodiment shown in Fig. 1.
FIG. 3 is a top view of the embodiment shown in Fig. 1.
FIG. 4 is a back view of the embodiment shown in Fig. 1.
FIG. 5 is an exploded view of a second embodiment.
FIG. 6 is a top view of the second embodiment shown in Fig 5.
FIG. 7 is a back view of the second embodiment shown in Fig 5.

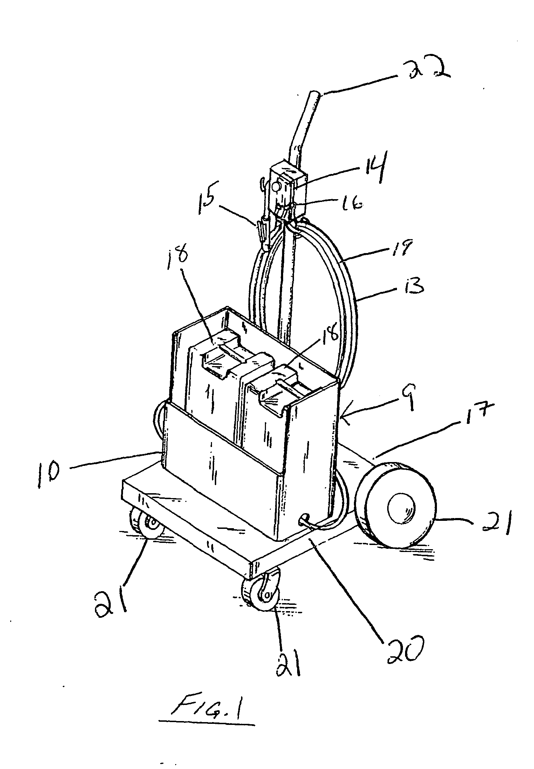

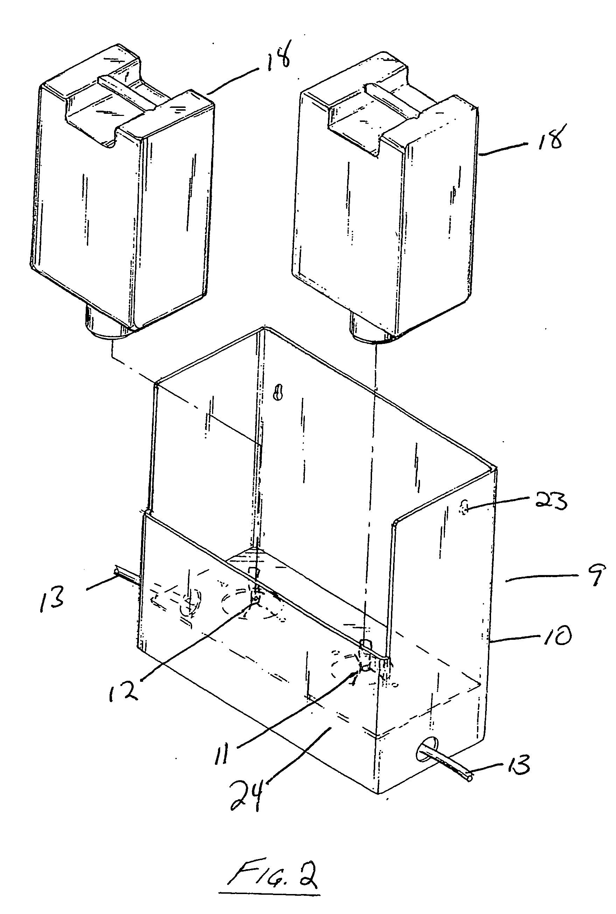

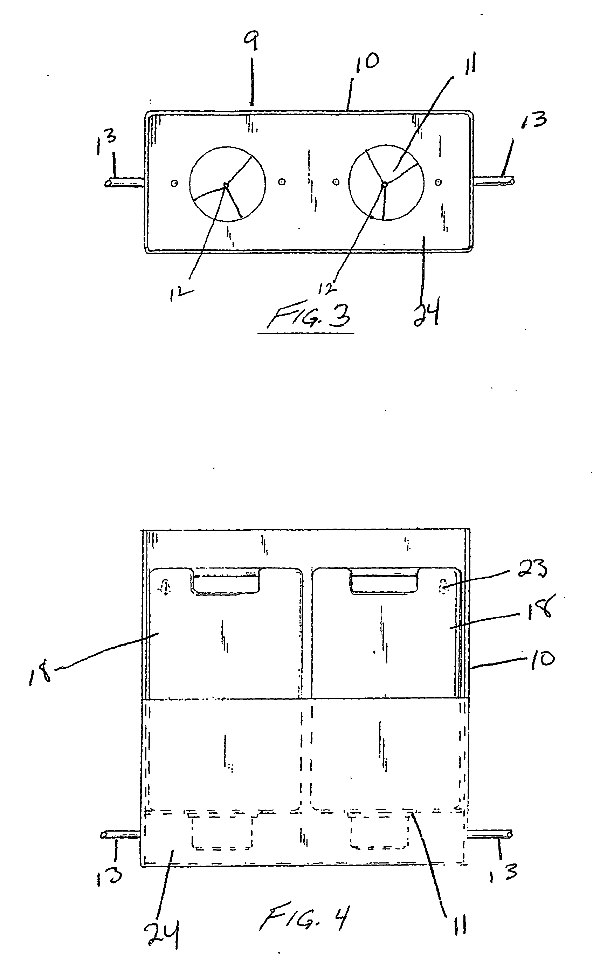

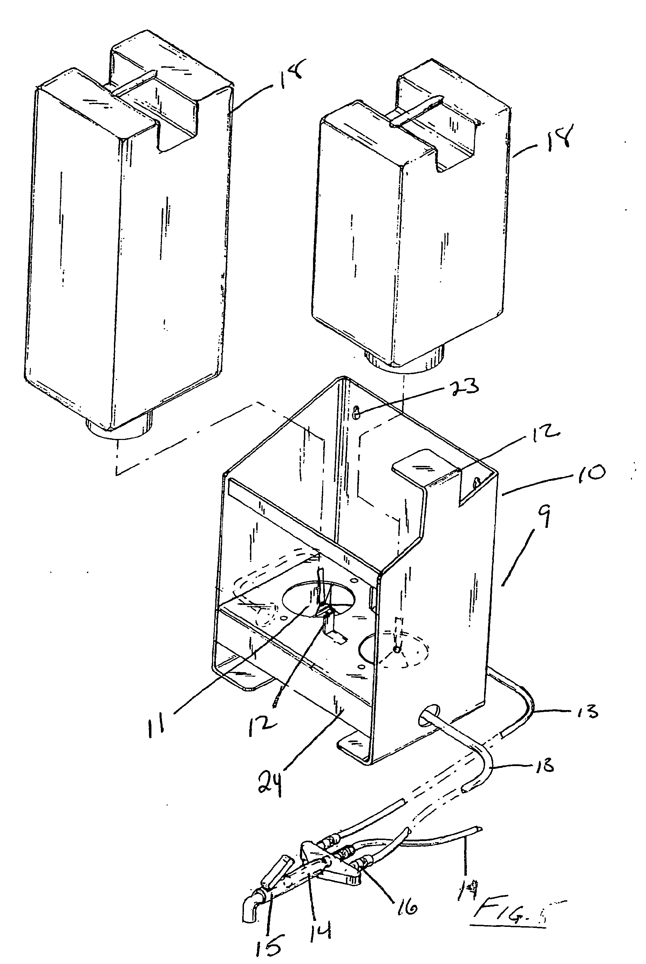

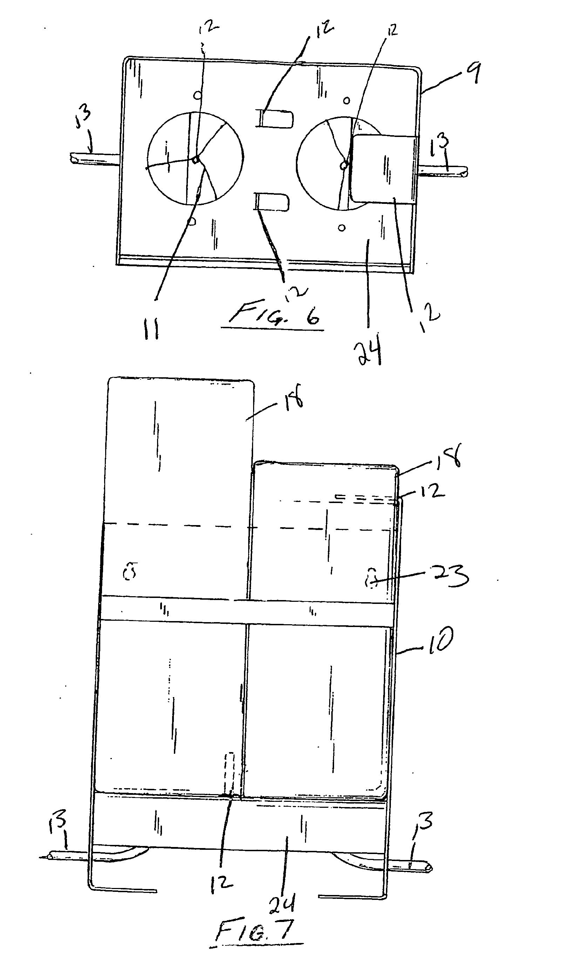

The embodiments of Fig. 1-7 are provided for illustrative purposes and do not form part of the invention.

### DETAILED DESCRIPTION

The invention comprises a multi component chlorine dioxide producing sanitizing and disinfecting composition as defined in claim 1 . The preferred solvent is water. The invention further includes a diluent, the preferred diluent is water. The activator of the current invention is a component which reduces the pH levels to 5 or lower which aids in the rapid reaction rate in order to produce effective amounts of chlorine dioxide. The preferred embodiment of the activator in the invention is an acid with the most preferred of embodiment being phosphoric acid. The preferred chlorite and chloride are an alkali metal chlorite and an alkali metal chloride respectfully. The most preferred chlorite and chloride are sodium chlorite and sodium chloride respectively. The chlorite, the activator and the chloride are in amounts which will produce an effective quantity of chlorine dioxide in less than 5 minutes and preferably in less than 3 minutes.

The invention produces effective amounts of chlorine dioxide when the chloride, the chlorite and the activator are present in the preferred amounts as follows; the chloride is less than 3,500 ppm (as sodium chloride), the chlorite is less than 100 ppm (as sodium chlorite) and the activator is less than 5,250 ppm. The invention further produces an effective amount of chlorine dioxide if the chloride to chlorite is in a molar ratio of at least 20:1 respectively.

The invention further requires that certain components must be stored separately in order to prevent the reaction from occurring before desired. The activator and the chlorite must be separated, but the chloride may be combined with either the chlorite or the activator, thus simplifying the system to two components.

### EXAMPLES

The data contained in Tables 1-5 correspond to the system based on chlorite (sodium chlorite), an activator (phosphoric acid) and a chloride salt (sodium chloride).

**Table 1: % Yield of Chlorine Dioxide vs Molar Ratio of NaCl to NaClO₂ at fixed NaClO₂ concentration of 0.030 gm/l (30 ppm)**

| [H₃PO₄] | [NaCl] | [NaClO₂] | [ClO₂] | Molar | yield |
|---|---|---|---|---|---|
| gm/l | gm/l | gm/l | ppm | Ratio | % |
| (as 75% active technical material) | (as 100% active) | (as 100% active) | (at 3 minutes) | [NaCl]:[NaClO₂] | based on stoichiometry |
| 4.31 | 0.00 | 0.030 | 1.0 | 0 : 1 | 4% |
| 4.31 | 0.74 | 0.030 | 3.0 | 38 : 1 | 13% |
| 4.31 | 1.10 | 0.030 | 8.0 | 57 : 1 | 36% |
| 4.31 | 1.45 | 0.030 | 14.6 | 75 : 1 | 65% |
| 4.31 | 1.74 | 0.030 | 13.7 | 90 : 1 | 61% |
| 4.31 | 2.26 | 0.030 | 12.8 | 117 : 1 | 57% |

**Table 2: % Yield of Chlorine Dioxide vs Molar Ratio of NaCl to NaClO₂ at fixed NaClO₂ concentration of 0.045 gm/l (45 ppm)**

| [H₃PO₄] | [NaCl] | [NaClO₂] | [ClO₂] | Molar | yield |
|---|---|---|---|---|---|
| gm/l | gm/l | gm/l | ppm | Ratio | % |
| (as 75% active technical material) | (as 100% active) | (as 100% active) | (at 3 minutes) | [Nacl]:[NaClO₂] | based on stoichiometry |
| 4.31 | 1.10 | 0.045 | 6.4 | 38 : 1 | 19% |
| 4.31 | 1.45 | 0.045 | 18.7 | 50 : 1 | 56% |
| 4.31 | 1.74 | 0.045 | 19.3 | 60 : 1 | 58% |
| 4.31 | 2.26 | 0.045 | 21.8 | 78 : 1 | 65% |
| 4.31 | 2.96 | 0.045 | 21.4 | 102 : 1 | 64% |
| 4.31 | 3.48 | 0.045 | 21.1 | 120 : 1 | 63% |

**Table 3: % Yield of Chlorine Dioxide vs Molar Ratio of NaCl to NaClO₂ at fixed NaClO₂ concentration of 0.060 gm/l (60 ppm)**

| [H₃PO₄] | [NaCl] | [NaClO₂] | [ClO₂] | Molar | yield |
|---|---|---|---|---|---|
| gm/l | gm/l | gm/l | ppm | Ratio | % |
| (as 75% active technical material) | (as 100% active) | (as 100% active) | (at 3 minutes) | [NaCl]:[NaClO₂] | based on stoichiometry |
| 4.31 | 0.00 | 0.060 | 1.3 | 0 : 1 | 3% |
| 4.31 | 0.37 | 0.060 | 0.8 | 10 : 1 | 2% |
| 4.31 | 1.45 | 0.060 | 13.0 | 38 : 1 | 29% |
| 4.31 | 1.91 | 0.060 | 26.0 | 49 : 1 | 58% |
| 4.31 | 2.26 | 0.060 | 29.4 | 58 : 1 | 66% |
| 4.31 | 2.96 | 0.060 | 30.0 | 77 : 1 | 67% |
| 4.31 | 3.48 | 0.060 | 26.0 | 90 : 1 | 58% |

**Table 4: % Yield of Chlorine Dioxide vs Molar Ratio of NaCl to NaClO₂ at fixed NaCl concentration of 1.45 gm/l (1450 ppm)**

| [H₃PO₄] | [NaCl] | [NaClO₂] | [ClO₂] | Molar | yield |
|---|---|---|---|---|---|
| gm/l | gm/l | gm/l | Ppm | Ratio | % |
| (as 75% active technical material) | (as 100% active) | (as 100% active) | (at 3 minutes) | [NaCl]:[NaClO₂] - | based on stoichiometry |
| 4.31 | 1.45 | 0.018 | 6.3 | 125 : 1 | 47% |
| 4.31 | 1.45 | 0.030 | 14.6 | 75 : 1 | 65% |
| 4.31 | 1.45 | 0.045 | 18.7 | 50 : 1 | 56% |
| 4.31 | 1.45 | 0.060 | 13.0 | 38 : 1 | 29% |
| 4.31 | 1.45 | 0.068 | 10.8 | 33 : 1 | 21% |
| 4.31 | 1.45 | 0.090 | 5.7 | 25 : 1 | 9% |
| 4.31 | 1.45 | 0.153 | 0.8 | 15 : 1 | 1% |

**Table 5: % Yield of Chlorine Dioxide vs Phosphoric Acid Concentration at fixed NaCl and NaClO₂ concentrations of 1.45 gm/l (1450 ppm) and 0.045 gm/l (45 ppm) respectively**

| [HₐPO₄] | [NaCl] | [NaClP₂] | [ClO₂] | Molar | yield |
|---|---|---|---|---|---|
| gm/l | gm/l | gm/l | Ppm | Ratio | % |
| (as 75% active technical material) | (as 100% active) | (as 100% active) | (at 3 minutes) | [NaCl]:[NaClO₂] | based on stoichiometry |
| 0.00 | 1.45 | 0.045 | 0.0 | 50 : 1 | 0% |
| 0.54 | 1.45 | 0.045 | 10.1 | 50 : 1 | 31% |
| 1,08 | 1.45 | 0.045 | 15.3 | 50 : 1 | 46% |
| 2.18 | 1.45 | 0.045 | 14.7 | 50 : 1 | 44% |
| 3.27 | 1.45 | 0.045 | 16.7 | 50 : 1 | 50% |
| 4.31 | 1.45 | 0.045 | 18.7 | 50 : 1 | 56% |
| 5.23 | 1.45 | 0.045 | 18.0 | 50 : 1 | 54% |

**Table 6: Generation of Chlorine Dioxide vs Reaction Time**

| | Chloride approach | | |
|---|---|---|---|
| [H₃PO₄] gm/l (as 75% active technical material) [NaCl] gm/l (as 100% active) | 4.31 | 4.31 | 4.31 |
| | 1.45 | 1.10 | 0.75 |
| | | | |
| [NaClO₂] gm/l | 0.030 | 0.030 | 0.030 |
| (as 100% active) Molar ratio NaCl:NaClO₂ Molar ratio NaClO₂:Kl | 75:1 | 57:1 | 39:1 |
| | | | |

| reaction time (sec) | Chlorine | Dioxide | concentration (ppm) |
|---|---|---|---|
| 0 | 0.0 | 0.0 | 0.0 |
| 30 | 8.7 | 4.9 | 2.2 |
| 60 | 11.4 | 6.0 | 2.7 |
| 90 | | | |
| 120 | 15.0 | 7.0 | 3.1 |
| 150 | | | |
| 180 | 16.6 | 7.3 | 3.2 |
| 210 | | | |
| 240 | 17.6 | 7.4 | 3.3 |
| 270 | | | |
| 300 | 18.2 | 7.5 | 3.3 |

The relatively consistent production of chlorine dioxide, in terms of absolute concentration produced, is surprising and indicates that the method of the present invention is very robust. Thus, the method can withstand relatively large variation in relative dilution ratios of the two components and still produce a consistent concentration of chlorine dioxide.

The data presented clearly indicates that a finite but relatively very short reaction time is required to produce chlorine dioxide in the present method. This is a great benefit The relatively short reaction time means that the components can be combined by co-eduction or by simultaneously dispensing them together into water which is being or may be directed onto a surface to be treated with chlorine dioxide. The components are mixed and begin reacting as they are being directed onto the surface. Thus chlorine dioxide is generated in situ on the surface to be treated. There is therefore relatively little chlorine dioxide present in the stream as it is directed onto the surface and thus loss of chlorine dioxide to volatilization during spraying becomes virtually a non-issue.

Referring to Figs. 1-7, these Figures show for illustrative purposes dispensing apparatus generally 9 for combining each component of the multi component chlorine dioxide composition to dispense a single end product. The dispensing apparatus 9 includes a support member 10 supporting a connection member 11 including a locking member 12 with two supply members 13 and a diluent supply member 19 engaged with an dosing member 14 that is connected with a dispensing member 15.

An embodiment of the dispensing apparatus 9 further includes a cart member 17 to allow the dispensing apparatus 9 to be mobile rather than stationary. The cart member 17 includes a platform member 20 and wheels 21 as well as a handle member 22.

Another embodiment of the dispensing apparatus 9 has at least one connection member 11 arranged to engage at least one container member 18 and a locking member 12 to secure at least one container member 18 to the support member 10. The locking member 12 and the connecting member 11 are attached to the support member 10. The locking member 12 secures the container member 18 preventing the container member 18 from disengaging or slipping out of the dispensing apparatus 9. A supply member 13 is attached to at least one container member 18 via the support member 10. The supply member 13 is engaged with at least one metering tip 16 which is engaged with a dosing member 14 in fluid communication with the dispensing member 15. The liquid supply member 19 provides a diluent to the dosing member 14.

The dispensing apparatus 9 has at least one attachment member 23 to secure the dispenser 9 to a surface. The dispensing apparatus 9 may have two or more container members with corresponding connector members 11 that are connected to the support member 10 which is engaged with a base member 24. The dispensing apparatus 9 will have liquid supply members 13 engaged with the base member 24. The liquid supply members are attached to at least one metering tip 16 that connect to at least one dosing member 14 which is in communication with a dispensing member 15.

The aforementioned embodiments are the preferred embodiments and are not meant in any way to limit the scope of the invention as defined by the appended claims.

## Claims

1. A multi component chlorine dioxide producing sanitizing and disinfecting composition comprising:
a. a chlorite present in an amount of less than 100 ppm;
b. an activator;
c. a chloride salt;
d. a solvent; and
e. a diluent.

2. The multi component chlorine dioxide producing sanitizing and disinfecting composition of claim 1 wherein the solvent is water.

3. The multi component chlorine dioxide producing sanitizing and disinfecting composition of claim 1 wherein the diluent is water.

4. The multi component chlorine dioxide producing sanitizing and disinfecting composition of claim 1 wherein the activator is an acid.

5. The multi component chlorine dioxide producing sanitizing and disinfecting composition of claim 1 wherein the activator is phosphoric acid.

6. The multi component chlorine dioxide producing sanitizing and disinfecting composition of claim 1 wherein the chlorite is an alkali metal chlorite.

7. The multi component chlorine dioxide producing sanitizing and disinfecting composition of claim 1 wherein the chlorite is sodium chlorite.

8. The multi component chlorine dioxide producing sanitizing and disinfecting composition of claim 1 wherein the chloride is an alkali metal chloride.

9. The multi component chlorine dioxide producing sanitizing and disinfecting composition of claim 1 wherein the chloride is sodium chloride.

10. The multi component chlorine dioxide producing sanitizing and disinfecting composition of claim 1 wherein the components are in amounts to produce an effective quantity of chlorine dioxide in less than five minutes.

11. The multi component chlorine dioxide producing sanitizing and disinfecting composition of claim 1 wherein the chloride is less than 3500 ppm.

12. The multi component chlorine dioxide producing sanitizing and disinfecting composition of claim 1 wherein the activator is less than 5250 ppm.

13. The multi component chlorine dioxide producing sanitizing and disinfecting composition of claim 1 wherein the molar ratio of the chloride to chlorite is at least 20 to 1.

14. The multi component chlorine dioxide producing sanitizing and disinfecting composition of claim 1 including a surfactant in effective amounts to dean a surface or system.

15. The multi component chlorine dioxide producing sanitizing and disinfecting composition of claim 14 wherein the surfactant has biocidal attributes.

16. A method of producing chlorine dioxide with a multi component chlorine dioxide producing sanitizing and disinfecting composition of claim 1 wherein the chlorite, the activator and a chloride are stored separately and mixed immediately before use to produce a chlorine dioxide in effective amounts in less than 5 minutes.

17. A method of producing chlorine dioxide with a multi component chlorine dioxide producing sanitizing and disinfecting composition of claim 1 wherein the chlorite, the activator and the chloride salt are stored in at least two separate areas and passes through metering tips to an eductor wherein the separate components and a diluent stream are combined to form a predetermined concentration to be applied to a surface or system.

## Patentansprüche

1. Eine Chlordioxid erzeugende, reinigende und desinfizierende Mehrkomponentenzusammensetzung, umfassend:
a) ein Chlorit, das in einer Menge von weniger als 100 ppm vorliegt,
b) ein Aktivierungsmittel,
c) ein Chloridsalz,
d) ein Lösungsmittel und
e) ein Verdünnungsmittel.

2. Die Chlordioxid erzeugende, reinigende und desinfizierende Mehrkomponentenzusammensetzung gemäß Anspruch 1, wobei das Lösungsmittel Wasser ist.

3. Die Chlordioxid erzeugende, reinigende und desinfizierende Mehrkomponentenzusammensetzung gemäß Anspruch 1, wobei das Verdünnungsmittel Wasser ist.

4. Die Chlordioxid erzeugende, reinigende und desinfizierende Mehrkomponentenzusammensetzung gemäß Anspruch 1, wobei das Aktivierungsmittel eine Säure ist.

5. Die Chlordioxid erzeugende, reinigende und desinfizierende Mehrkomponentenzusammensetzung gemäß Anspruch 1, wobei das Aktivierungsmittel Phosphorsäure ist.

6. Die Chlordioxid erzeugende, reinigende und desinfizierende Mehrkomponentenzusammensetzung gemäß Anspruch 1, wobei das Chlorit ein Alkalimetallchlorit ist.

7. Die Chlordioxid erzeugende, reinigende und desinfizierende Mehrkomponentenzusammensetzung gemäß Anspruch 1, wobei das Chlorit Natriumchlorit ist.

8. Die Chlordioxid erzeugende, reinigende und desinfizierende Mehrkomponentenzusammensetzung gemäß Anspruch 1, wobei das Chlorid ein Alkalimetallchlorid ist.

9. Die Chlordioxid erzeugende, reinigende und desinfizierende Mehrkomponentenzusammensetzung gemäß Anspruch 1, wobei das Chlorid Natriumchlorid ist.

10. Die Chlordioxid erzeugende, reinigende und desinfizierende Mehrkomponentenzusammensetzung gemäß Anspruch 1, wobei die Komponenten in Mengen vorliegen, um eine wirksame Menge an Chlordioxid in weniger als fünf Minuten zu erzeugen.

11. Die Chlordioxid erzeugende, reinigende und desinfizierende Mehrkomponentenzusammensetzung gemäß Anspruch 1, wobei weniger als 3500 ppm des Chlorids vorliegt.

12. Die Chlordioxid erzeugende, reinigende und desinfizierende Mehrkomponentenzusammensetzung gemäß Anspruch 1, wobei weniger als 5250 ppm des Aktivierungsmittels vorliegt.

13. Die Chlordioxid erzeugende, reinigende und desinfizierende Mehrkomponentenzusammensetzung gemäß Anspruch 1, wobei das molare Verhältnis von Chlorid zu Chlorit wenigstens 20 zu 1 beträgt.

14. Die Chlordioxid erzeugende, reinigende und desinfizierende Mehrkomponentenzusammensetzung gemäß Anspruch 1, enthaltend ein Tensid in wirksamen Mengen zur Reinigung einer Oberfläche oder eines Systems.

15. Die Chlordioxid erzeugende, reinigende und desinfizierende Mehrkomponentenzusammensetzung gemäß Anspruch 14, wobei das Tensid biozide Eigenschaften aufweist.

16. Ein Verfahren zur Herstellung von Chlordioxid mit einer Chlordioxid erzeugenden, reinigenden und desinfizierenden Mehrkomponentenzusammensetzung gemäß Anspruch 1, wobei das Chlorit, das Aktivierungsmittel und ein Chlorid getrennt gelagert und unmittelbar vor der Verwendung gemischt werden, um Chlordioxid in wirksamer Menge in weniger als 5 Minuten zu erzeugen.

17. Ein Verfahren zur Herstellung von Chlordioxid mit einer Chlordioxid erzeugenden, reinigenden und desinfizierenden Mehrkomponentenzusammensetzung gemäß Anspruch 1, wobei das Chlorit, das Aktivierungsmittel und das Chloridsalz in zumindest zwei getrennten Bereichen gelagert und durch Dosierstücke zu einem Eduktor geleitet werden, in dem die separaten Komponenten und ein Verdünnungsstrom kombiniert werden, um eine vorbestimmte, auf eine Oberfläche oder ein System aufzubringende bzw. anzuwendende Konzentration auszubilden.

## Revendications

1. Composition multiconstituante d'hygiène et de désinfection produisant du dioxyde de chlore comprenant :
a. un chlorite présent dans une quantité inférieure à 100 ppm ;
b. un activateur ;
c. un sel de chlorure ;
d. un solvant ; et
e. un diluant.

2. Composition multiconstituante d'hygiène et de désinfection produisant du dioxyde de chlore selon la revendication 1, dans laquelle le solvant est l'eau.

3. Composition multiconstituante d'hygiène et de désinfection produisant du dioxyde de chlore selon la revendication 1, dans laquelle le diluant est l'eau.

4. Composition multiconstituante d'hygiène et de désinfection produisant du dioxyde de chlore selon la revendication 1, dans laquelle l'activateur est un acide.

5. Composition multiconstituante d'hygiène et de désinfection produisant du dioxyde de chlore selon la revendication 1, dans laquelle l'activateur est l'acide phosphorique.

6. Composition multiconstituante d'hygiène et de désinfection produisant du dioxyde de chlore selon la revendication 1, dans laquelle le chlorite est un chlorite de métal alcalin.

7. Composition multiconstituante d'hygiène et de désinfection produisant du dioxyde de chlore selon la revendication 1, dans laquelle le chlorite est le chlorite de sodium.

8. Composition multiconstituante d'hygiène et de désinfection produisant du dioxyde de chlore selon la revendication 1, dans laquelle le chlorure est un chlorure de métal alcalin.

9. Composition multiconstituante d'hygiène et de désinfection produisant du dioxyde de chlore selon la revendication 1, dans laquelle le chlorure est le chlorure de sodium.

10. Composition multiconstituante d'hygiène et de désinfection produisant du dioxyde de chlore selon la revendication 1, dans laquelle les constituants sont présents dans des quantités pour produire une quantité efficace de dioxyde de chlore en moins de cinq minutes.

11. Composition multiconstituante d'hygiène et de désinfection produisant du dioxyde de chlore selon la revendication 1, dans laquelle le chlorure est présent à moins de 3 500 ppm.

12. Composition multiconstituante d'hygiène et de désinfection produisant du dioxyde de chlore selon la revendication 1, dans laquelle l'activateur est présent à moins de 5 250 ppm.

13. Composition multiconstituante d'hygiène et de désinfection produisant du dioxyde de chlore selon la revendication 1, dans laquelle le rapport molaire du chlorure au chlorite est d'au moins 20 à 1.

14. Composition multiconstituante d'hygiène et de désinfection produisant du dioxyde de chlore selon la revendication 1 comprenant un tensioactif dans des quantités efficaces pour nettoyer une surface ou un système.

15. Composition multiconstituante d'hygiène et de désinfection produisant du dioxyde de chlore selon la revendication 14, dans laquelle le tensioactif présente des propriétés biocides.

16. Procédé de production de dioxyde de chlore avec une composition multiconstituante d'hygiène et de désinfection produisant du dioxyde de chlore selon la revendication 1, dans lequel le chlorite, l'activateur et un chlorure sont stockés séparément et mélangés immédiatement avant l'utilisation pour produire un dioxyde de chlore dans des quantités efficaces en moins de 5 minutes.

17. Procédé de production de dioxyde de chlore avec une composition multiconstituante d'hygiène et de désinfection produisant du dioxyde de chlore selon la revendication 1, dans lequel le chlorite, l'activateur et le sel de chlorure sont stockés dans au moins deux zones séparées et passent à travers des pointes de dosage vers un éjecteur dans lequel les constituants séparés et un courant de diluant sont combinés pour former une concentration prédéterminée à appliquer sur une surface ou un système.
